## Europäisches Patentamt
## European Patent Office
## Office européen des brevets

(19)

(11) Veröffentlichungsnummer: **0 033 466**
**B1**

(12)

# EUROPÄISCHE PATENTSCHRIFT

(45) Veröffentlichungstag der Patentschrift:
**30.11.83**

(51) Int. Cl.³: **A 61 B 6/02,** G 01 T 1/29

(21) Anmeldenummer: **81100389.6**

(22) Anmeldetag: **20.01.81**

(54) **Strahlendiagnostikgerät.**

(30) Priorität: **30.01.80 DE 3003295**

(43) Veröffentlichungstag der Anmeldung:
**12.08.81 Patentblatt 81/32**

(45) Bekanntmachung des Hinweises auf die Patenterteilung:
**30.11.83 Patentblatt 83/48**

(84) Benannte Vertragsstaaten:
**DE FR GB**

(56) Entgegenhaltungen:
**FR - A - 2 295 420**
**FR - A - 2 335 854**
**FR - A - 2 352 311**
**GB - A - 2 005 405**
**GB - A - 2 007 457**
**US - A - 4 153 839**
**US - A - 4 159 424**

(73) Patentinhaber: **SIEMENS AKTIENGESELLSCHAFT,
Berlin und München Wittelsbacherplatz 2,
D-8000 München 2 (DE)**

(72) Erfinder: **Conrad, Bernhard, Dipl.-Phys., Rosenau 19,
D-8520 Erlangen (DE)**

## Strahlendiagnostikgerät

Die Erfindung betrifft ein Strahlendiagnostikgerät gemäß dem Oberbegriff des Patentanspruchs 1.

Ein Strahlendiagnostikgerät dieser Art kann dazu verwendet werden, eine Transversalschicht eines Patienten unter verschiedenen Projektionen abzutasten und aus den elektrischen Ausgangssignalen des Detektors bzw. der Detektoren die Schwächungskoeffizienten vorbestimmter Bildpunkte der untersuchten Transversalschicht zu berechnen und bildlich wiederzugeben. Ein Gerät dieser Art ist ein sog. Computertomograph.

Für ausreichende elektrische Ausgangssignale des Halbleiterdetektors bzw. der Halbleiterdetektoren ist in dem Fall, in dem der Winkel zwischen der Strahleneintrittsfläche des Detektors und der Strahlung 90° beträgt, eine relativ große Schichtstärke erforderlich, so daß ausreichend viele Strahlenquanten absorbiert werden. Derartig große Schichtdicken sind jedoch in der Praxis bei verschiedenen Materialien nicht zu realisieren. Zur Lösung dieses Problems ist es bekannt, mehrere plattenförmige Halbleiterdetektoren zur Erfassung der Strahlung auf einem Strahlenweg hintereinander anzuordnen und durch Leuchtschichten voneinander zu trennen (DE-A-2 622 655). Dieser Vorschlag bedingt jedoch bei der Verwendung einer Vielzahl in Reihe liegender Detektoren zur Erfassung eines fächerförmigen Strahlenbündels einen sehr aufwendigen Aufbau.

Durch die US-A-4 159 424 ist beschrieben, daß bei einer Detektoranordnung für Röntgenstrahlung die Abmessungen des Detektors die Strahlenabsorption bestimmen. Es werden ferner Fehler der Intensitätsmessung untersucht, die von einer Desorientierung des Detektors bei Einfallswinkeln in der Nähe von 0° herrühren.

Ferner ist in der GB-A-2 007 457 ein Computertomograph beschrieben, bei dem ein Detektorring vorhanden ist, innerhalb dessen eine Röntgenstrahlenquelle um das Aufnahmeobjekt kreist, welche ein fächerförmiges Röntgenstrahlenbündel aussendet. Aufgrund der konstruktiven Ausbildung dieses Computertomographen bildet die Strahleneintrittsfläche jedes Detektors mit der Strahlung einen Einfallswinkel der zwischen 0° und 45° liegt.

Der Erfindung liegt die Aufgabe zugrunde, ein Strahlendiagnostikgerät gemäß dem Oberbegriff des Patentanspruchs 1 zu schaffen, um mit einem einzigen plattenförmigen Detektor pro Strahlenweg die Strahlenabsorption gegenüber dem Stand der Technik zu verbessern.

Diese Aufgabe ist erfindungsgemäß durch die im Kennzeichen des Patentanspruchs 1 angegebene Ausbildung gelöst. Die Strahleneintrittsfläche des Halbleiterdetektors ist also bei dem erfindungsgemäßen Strahlendiagnostikgerät gegenüber der zu erfassenden Strahlung so geneigt, daß der Absorptionswert gegenüber dem Stand der Technik vergrößert wird. Besonders zweckmäßig ist es dabei, den Einfallswinkel zwischen 80° und 88° zu legen. Der Einfallswinkel ist definitionsgemäß der von der einfallenden Strahlung mit der Flächennormalen der Strahleneintrittsfläche gebildete Winkel. Der Detektor kann ein Halbleiterdetektor aber auch eine Kombination einer Leuchtstoffschicht bzw. eines Szintillationskristalls mit einem Halbleiterdetektor sein.

Eine besonders zweckmäßige Ausgestaltung der Erfindung ergibt sich aus dem Patentanspruch 4. Diese Anordnung eignet sich besonders als Strahlenempfänger in einem Computertomographen.

Die Erfindung ist nachfolgend anhand mehrerer, in der Zeichnung dargestellter Ausführungsbeispiele näher erläutert. Es zeigt:

Fig. 1 eine Darstellung der für die Erfindung wesentlichen Teile eines Stahlendiagnostikgerätes nach der Erfindung,

Fig. 2 einen vergrößerten Ausschnitt aus der Figur 1,

Fig. 3 eine andere Ausführungsform der erfindungswesentlichen Teile eines Strahlendiagnostikgerätes nach der Erfindung, und

Fig. 4 eine Variante der in der Figur 3 dargestellten Ausführung.

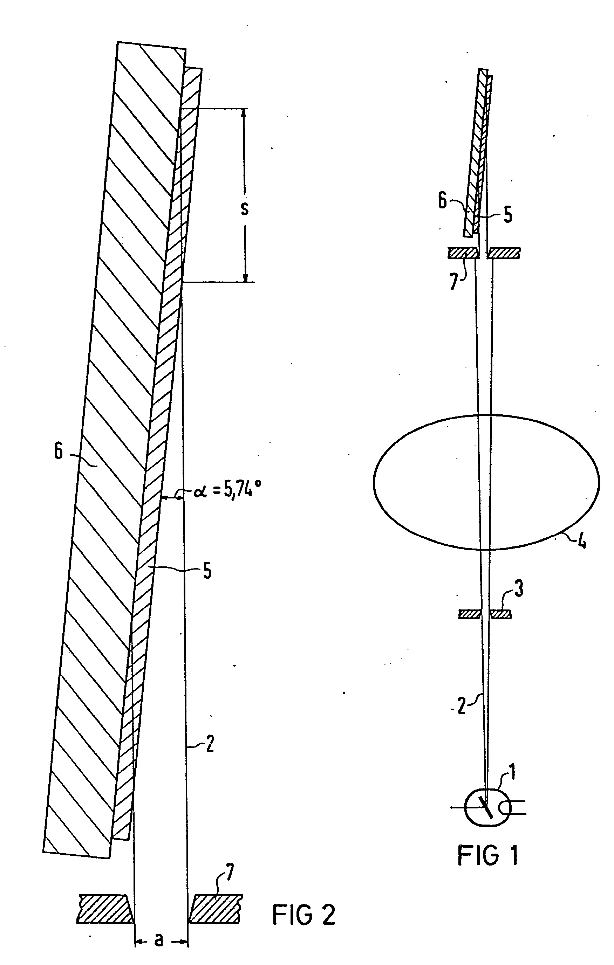

In der Figur 1 ist eine Röntgenröhre 1 als Strahlenquelle dargestellt, von deren Röntgenstrahlenbündel mittels einer Primärstrahlenblende 3 ein schmales Strahlenbündel 2 eingeblendet wird, das eine Transversalschicht eines Patienten 4 durchdringt. Die aus dem Patienten 4 austretende Strahlung trifft auf einem plattenförmigen Halbleiterdetektor 5, beispielsweise aus Selen, auf, welcher auf einer plattenförmigen Maßelektrode 6 angeordnet ist. Unmittelbar vor dem Halbleiterdetektor 5 liegt eine Blende 7, die die Strahlung auf ein Maß einblendet, bei dem eine Überstrahlung des Halbleiterdetektors 5 verhindert ist.

Der Halbleiterdetektor 5 erzeugt ein elektrisches Signal, das von der empfangenen Strahlungsintensität abhängt. Die in der Figur 1 dargestellte Anordnung kann dazu dienen, eine Transversalschicht des Patienten 4 unter verschiedenen Projektionen abzutasten und aus den Ausgangssignalen des Halbleiterdetektors 5 ein Bild der untersuchten Transversalschicht in der bei Computertomographen bekannten Weise zu berechnen und wiederzugeben.

Aus der Figur 2 geht hervor, daß der Halbleiterdetektor 5 unter einem Winkel $\alpha$ von 5,74° gegenüber dem empfangenen Strahlenbündel 2 geneigt ist (Glanzwinkel). Dieser Winkel ergibt eine Verlängerung des Absorptionsweges s für die Strahlung im Halbleiterdetektor 5 gegenüber dem Fall, in dem der Winkel 90° beträgt, um den Faktor 10. Bei einer Schichtdicke des Halbleiterdetektors 5 von 0,5 mm ist also die für die Absorption der Strahlung wirksame Schichtdicke 5 mm. Diese Schichtdicke reicht aus, damit praktisch 100% der Strahlung absorbiert werden. Dies gilt z. B. für eine Röhrenspannung von 70 kV. Bei einer Röhrenspannung von z. B. 125 kV benötigt man bei dem gleichen Winkel eine Schichtdicke des Halbleiters von 1 mm, um annähernd 100% Absorption zu erhalten. Im Rahmen der Erfindung sind selbstverständlich auch andere Glanzwinkel im Bereich zwischen 0° und 45°, insbesondere zwischen 20° und 1°, vorzugsweise zwischen 10° und 2°, sinnvoll, so daß sich Einfallswinkel zwischen 70° und 89° bzw. 80° und 88° ergeben. Der Einfallswinkel ist dabei gleich der Differenz zwischen 90° und dem Glanzwinkel.

Aufgrund der in der Figur 2 dargestellten Schrägstellung des Halbleiterdetektors 5 gegenüber dem Strahlenbündel 2 und der damit verbundenen Absorption nahe 100% ist erreicht, daß eine geringfügige Änderung des Absorptionsweges s durch Änderung des Einfallswinkels aufgrund von Toleranzen eine praktisch vernachlässigbare Änderung der Strahlenabsorption bewirkt.

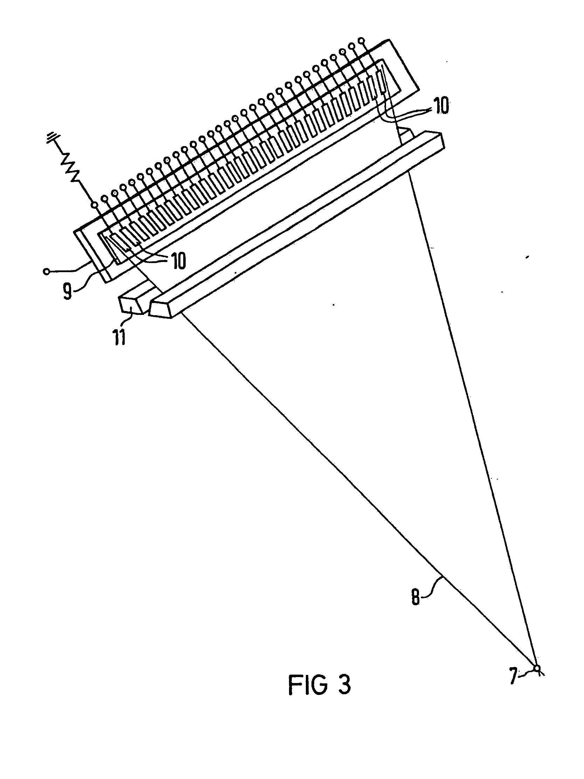

In der Figur 3 ist eine Anordnung gezeigt, bei der der Strahlenempfänger für die von einem Fokus 7 einer Röntgenröhre ausgehende, fächerförmige Röntgenstrahlung 8 eine plattenförmige, gemeinsame Elektrode mit einer gemeinsamen Halbleiterschicht 9 aufweist, auf der mehrere Kontaktflächen 10 reihenförmig aufgebracht sind. Die gemeinsame Elektrode mit der Halbleiterschicht 9 ist gegenüber der Strahlung 8 um den Einfallswinkel geneigt, der zum Rand hin geringfügig zunimmt. Bei dieser Anordnung ist die gleichzeitige Erzeugung einer Vielzahl von Meßwerten möglich. Auch hier liegt vor dem Strahlenempfänger 9, 10 eine Blende 11, die den wirksamen Detektorquerschnitt definiert (Maß a in Figur 2).

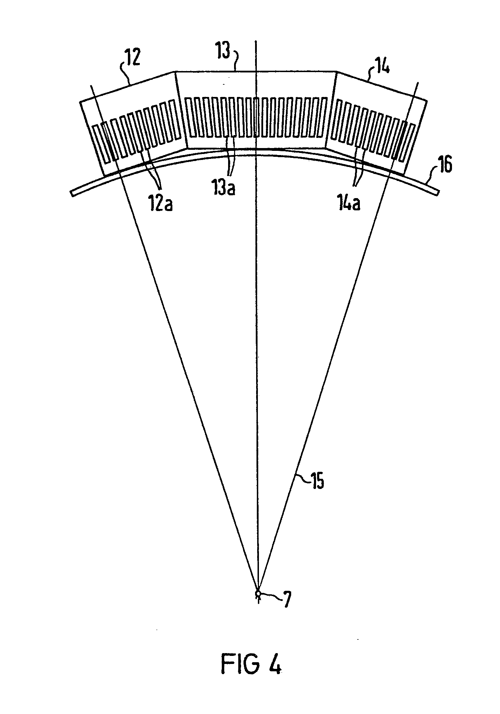

Für einen Computertomographen ist ein Strahlenempfänger sinnvoll, bei dem eine gemeinsame, langgestreckte Elektrode als Teil eines Kegelmantels ausgebildet ist, auf der mehrere Halbleiterdetektoren reihenförmig aufgebracht sind oder die eine durchgehende Halbleiterschicht trägt, die z. B. im Falle von Selen durch Aufdampfen erzeugt werden kann, und die gegenüber der Strahlung um den Glanzwinkel geneigt ist. Zur Annäherung einer solchen Anordnung sind gemäß Figur 4 drei plattenförmige gemeinsame Elektroden mit einer Halbleiterschicht 12 bis 14 vorgesehen, von denen jede gegenüber der Strahlung 15 um den Glanzwinkel geneigt ist und auf denen jeweils mehrere Kontaktflächen 12a bis 14a in einer Anordnung gemäß Figur 3 aufgebracht sind. Der Glanzwinkel nimmt dabei von der Mitte zum Rand der einzelnen Platten geringfügig zu.

Die gemeinsamen Elektroden 12 bis 14 sind dabei Teile der Seitenfläche einer Pyramide. Auch hier ist vor dem Strahlenempfänger ein Kollimator 16 angeordnet.

## Patentansprüche

1. Strahlendiagnostikgerät mit einer Strahlenquelle (1), die ein ein Aufnahmeobjekt (4) durchdringendes Strahlenbündel (2, 8, 15) erzeugt und einem Strahlenempfänger, der mindestens einen plattenförmigen Detektor (5, 9, 12, 13, 14) aufweist, dadurch gekennzeichnet, daß die Flächennormale zur Strahleneintrittsfläche des Detektors (5, 9, 12 bis 14) mit der Strahlung (2, 8, 15) einen Einfallswinkel bildet, der zwischen 45° und 90° liegt.

2. Strahlendiagnostikgerät nach Anspruch 1, dadurch gekennzeichnet, daß der Einfallswinkel zwischen 70° und 89° liegt.

3. Strahlendiagnostikgerät nach Anspruch 2, dadurch gekennzeichnet, daß der Einfallswinkel zwischen 80° und 88° liegt.

4. Strahlendiagnostikgerät nach einem der Ansprüche 1 bis 3, dadurch gekennzeichnet, daß der Strahlenempfänger (9, 10) eine für eine Halbleiterschicht (9) gemeinsame, plattenförmige Elektrode aufweist, wobei auf der gegenüberliegenden freien Fläche der Halbleiterschicht (9) mehrere Kontaktflächen (10) reihenförmig aufgebracht sind, und die integrale Elektroden-Halbleiter-Schicht gegenüber der Strahlung (8) um den Einfallswinkel geneigt ist.

5. Strahlendiagnostikgerät nach einem der Ansprüche 1 bis 4, dadurch gekennzeichnet, daß der Strahlenempfänger eine gemeinsame, langgestreckte Elektrode als Teil eines Kegelmantels aufweist, auf der mehrere Halbleiterdetektoren reihenförmig aufgebracht sind, und die gemeinsame Elektrode gegenüber der Strahlung um den Einfallswinkel geneigt ist.

6. Strahlendiagnostikgerät nach einem der Ansprüche 1 bis 3, dadurch gekennzeichnet, daß der Strahlenempfänger mehrere plattenförmige Elektroden (12 bis 14) aufweist, auf denen jeweils eine Halbleiterschicht aufgebracht ist, die mit einer Reihe von Kontaktflächen (12a bis 14a) versehen ist, daß die integralen Elektroden-Halbleiter-Schichten (12 bis 14) gegenüber der Strahlung (15) um den Einfallswinkel geneigt sind, und daß die integralen Elektroden-Halbleiter-Schichten Teile der Seitenflächen einer Pyramide sind.

## Claims

1. A radiation diagnostic device having a radiation source (1) which produces a bundle of rays (2, 8, 15) which penetrate an exposed object (4), and a radiation receiver which hat at least one plateshaped detector (5, 9, 12, 13, 14), characterised in that the surface of the detector normal to the radiation incident surface of the detector (5, 9, 12 to 14), forms with the radiation (2, 8, 15), an angle of incidence of between 45° and 90°.

2. A radiation diagnostic device as claimed in Claim 1, characterised in that the angle of incidence is between 70° and 89°.

3. A radiation diagnostic device as claimed in Claim 2, characterised in that the angle of incidence is between 80° and 88°.

4. A radiation diagnostic device as claimed in one of Claims 1 to 3, characterised in that the radiation receiver (9, 10) has a common plate-shaped electrode for a semiconductor layer (9), on the opposite exposed surface of which semiconductor layer (9), a plurality of contact surfaces (10) are arranged in a row, and the integral electrodesemiconductor layer is inclined relative to the radiation (8) by the angle of incidence.

5. A radiation diagnostic device as claimed in one of Claims 1 to 4, characterised in that the radiation receiver has a common elongated electrode forming part of a conical casing, on which a plurality of semiconcuctor detectors are aranged in a row; and that the common electrode is inclined relative to the radiation by the angle of incidence.

6. A radiation diagnostic device as claimed in one of Claims 1 to 3, characterised in that the radiation receiver has a plurality of plate- shaped electrodes (12 to 14) each of which has a semiconductor layer which is provided with a row of contact surfaces (12a to 14a); that the integral elektrode-semiconductor layers (12 to 14) are inclined relative to the radiation (15) by the angle of incidence; and that the integral electrode-semiconductor layers form parts of the lateral surfaces of a pyramid.

## Revendications

1. Appareil de radiodiagnostic comportant une source de rayons (1), qui produit un faisceau de rayons (2, 8, 15) qui traverse un objet de prise de vue (4), et un récepteur de rayons qui comporte au moins un détecteur en forme de plaquette (5, 9, 12, 13, 14), caractérisé par le fait que la normale à la surface de pénétration des rayons du détecteur (5, 9, 12 à 14) forme avec le rayonnement (2, 8, 15) un angle d'incidence qui se trouve entre 45° et 90°.

2. Appareil de radiodiagnostic suivant la revendication 1, caractérisé par le fait que l'angle d'incidence se trouve entre 70° et 89°.

3. Appareil de radiodiagnostic suivant la revendication 2, caractérisé par le fait que l'angle d'incidence se trouve entre 80° et 88°.

4. Appareil de radiodiagnostic suivant l'une des revendications 1 à 3, caractérisé par le fait que le récepteur de rayons (9, 10) comporte une électrode commune en forme de plaquette, pour une couche semiconductrice (9), plusieurs surfaces de contact (10) étant disposées en série sur la surface libre opposée de la couche semiconductrice (9), et l'ensemble de l'électrode et de la couche semiconductrice étant incliné de l'angle d'incidence par rapport au rayonnement (8).

5. Appareil de radiodiagnostic suivant l'une des revendications 1 à 4, caractérisé par le fait que le récepteur de rayons comporte une électrode allongée commune se présentant sous la forme d'une enveloppe conique, sur laquelle sont disposés en série plusieurs détecteurs à semiconducteurs, et que l'électrode commune est inclinée de l'angle d'incidence par rapport au rayonnement.

6. Appareil de radiodiagnostic suivant l'une des revendications 1 à 3, caractérisé par le fait que le récepteur de rayons comporte plusieurs électrodes en forme de plaquettes (12 à 14), sur lesquelles est respectivement disposée une couche semiconductrice qui est munie d'une série de surfaces de contact (12a à 14a), que les ensembles comprenant les électrodes et les couches semiconductrices (12 à 14) sont inclinés de l'angle d'incidence par rapport au rayonnement (15), et que les ensembles comprenant les électrodes et les couches semiconductrices font partie des surfaces latérales d'une pyramide.

S

6

α = 5,74°

5

2

7

FIG 2

a

6
5

7

4

3

2

1

FIG 1

FIG 3

FIG 4